(19) Europäisches Patentamt
European Patent Office
Office européen des brevets

(11) Numéro de publication : **0 069 648**
**B1**

# (12) FASCICULE DE BREVET EUROPÉEN

(45) Date de publication du fascicule du brevet : **12.02.86**

(21) Numéro de dépôt : **82401192.8**

(22) Date de dépôt : **28.06.82**

(51) Int. Cl.4 : **C 07 B 59/00**, C 07 C 51/363// C07C57/18, C07C33/042, C07C55/32, C07C53/19, C07C59/42, C07C59/01, C07C143/28, C07C57/52, A61K45/02

## (54) Procédé de préparation extemporanée d'un acide gras injectable, marqué à l'iode radioactif et préparation de dérivés iodes utilisables pour la mise en oeuvre de ce procédé.

(30) Priorité : **02.07.81 FR 8113039**

(43) Date de publication de la demande : **12.01.83 Bulletin 83/02**

(45) Mention de la délivrance du brevet : **12.02.86 Bulletin 86/07**

(84) Etats contractants désignés : **AT BE CH FR GB IT LI LU NL SE**

(56) Documents cités :
**FR-A- 2 419 265**
**JOURNAL OF NUCLEAR MEDICINE, vol. 16, no. 1, janvier 1975, New York (US), G.D. ROBINSON et al.: "Radioiodinated fatty acids for heart imaging: iodine monochloride addition compared with iodide replacement labeling", pages 17-21**

(73) Titulaire : **COMMISSARIAT A L'ENERGIE ATOMIQUE Etablissement de Caractère Scientifique Technique et Industriel**
**31/33, rue de la Fédération**
**F-75015 Paris (FR)**

(72) Inventeur : **Bardy, André**
**48, Les Genêts**
**F-91420 Morangis (FR)**
Inventeur : **Comet, Michel**
**7, Boulevard de Jomardière**
**F-38120 Saint Egrève (FR)**
Inventeur : **Coornaert, Sabine**
**7, rue des Sables**
**F-78720 Dampierre (FR)**
Inventeur : **Mathieu, Jean-Paul**
**67, Rivoire de La Dame**
**F-38360 Sassenage (FR)**
Inventeur : **Riche, Françoise**
**Lieu dit " La Penelle " Theys**
**F-38570 Goncelin (FR)**
Inventeur : **Vidal, Michel**
**Baratière Saint Nazaire Les Eymes**
**F-38330 Saint Ismier (FR)**

(74) Mandataire : **Mongrédien, André et al**
**c/o BREVATOME 25, rue de Ponthieu**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 069 648 B1

**Description**

La présente invention a pour objet un procédé de préparation extemporanée d'un acide gras injectable marqué en position ω à l'aide d'iode radioactif.

De façon plus précise, elle concerne la préparation d'acides gras marqués à l'iode 123 ou à l'iode 131, utilisables comme produits radio-pharmaceutiques pour étudier les troubles du métabolisme cardiaque chez l'homme par scintigraphie.

On sait que les acides gras à longue chaîne constituent l'une des principales sources d'énergie du muscle cardiaque et que le métabolisme d'acides gras marqués en position oméga par les isotopes radioactifs de l'iode : $^{123}I$ et $^{131}I$ s'est révélé très voisin de celui des acides gras naturels. Aussi, l'étude de leur captation myocardique permet d'avoir accès au fonctionnement cellulaire, et les acides gras marqués présentent de ce fait un grand intérêt en médecine nucléaire, d'autant plus qu'ils répondent par ailleurs aux critères qui doivent être remplis en vue d'une utilisation comme produits radio-pharmaceutiques. En effet, le marquage n'affecte pas les propriétés biologiques de la molécule ; le choix de l'iode radioactif 123 comme marqueur répond à des considérations biologiques et techniques telles que : période courte de 6 à 20 h et émission γ d'une énergie de l'ordre de 100 à 200 keV ; par ailleurs, les dérivés iodés d'acides gras sont peu toxiques et leur taux de captation est élevé.

Cependant leur utilisation dans les services hospitaliers pose certains problèmes car pour le moment il est impossible d'assurer la préparation du produit marqué, au moment de son utilisation, dans les laboratoires de médecine nucléaire.

Jusqu'à présent, pour obtenir des acides gras marqués par de l'iode radioactif, on a utilisé la méthode de Robinson qui consiste à échanger par de l'iode radioactif le brome de l'acide gras bromé à marquer (voir Int. J. Appl. Radiat. Isot. Vol. 28, P. 149, 1977). Généralement, on réalise la réaction d'échange sur l'acide gras bromé en solution dans la butanone auquel on a ajouté une solution d'iodures radioactifs ($Na^-$, $^{123}I^-$) dans le même solvant ; l'échange est réalisé en portant la solution au reflux pendant 90 minutes ou plus. Une étude préliminaire a montré que cette méthode conduit à des rendements de marquage peu reproductibles et très souvent inférieurs à 80 %. Cette technique, qui met en œuvre une suite d'opérations longues et relativement complexes, nécessite, compte tenu du faible rendement de l'échange, la purification avant injection du produit radiopharmaceutique.

Pour réaliser cette étape de purification de l'acide marqué, on utilise généralement la chromatographie HPLC, c'est-à-dire une technique qui ne peut être utilisée en routine dans un laboratoire de médecine nucléaire ne disposant pas d'un personnel spécialisé. De ce fait, il est impossible de préparer l'acide gras marqué dans le cadre du service hospitalier.

La présente invention a précisément pour objet un procédé de préparation d'un acide gras marqué à l'iode radioactif qui conduit à un rendement de marquage élevé et qui permet de ce fait d'éviter l'inconvénient des procédés antérieurs. En effet, compte tenu du rendement de marquage élevé, il n'est plus nécessaire de mettre en œuvre une étape de purification très délicate pour isoler le produit radiopharmaceutique avant son utilisation. Ainsi, la préparation de l'acide gras marqué peut être réalisée dans le laboratoire chaud d'un service de médecine nucléaire par un personnel non spécialisé.

Le procédé selon l'invention, de préparation d'un acide gras marqué à l'iode radioactif consiste à faire réagir un acide gras bromé ou iodé en position oméga avec de l'iodure radioactif pour échanger par de l'iode radioactif le brome ou l'iode dudit acide gras, se caractérise en ce que l'on utilise de l'iodure radioactif à l'état sec ou une solution aqueuse d'iodure radioactif et en ce que l'on réalise la réaction en présence d'un iodure entraîneur.

Généralement, on réalise cette réaction d'échange en chauffant pendant environ 10 min., à une température d'environ 102-105 °C, une solution de l'acide gras bromé ou iodé dans une cétone miscible à l'eau contenant l'iodure entraîneur constitué par exemple par un iodure métallique soluble dans une cétone tel que l'iodure de sodium, après avoir ajouté à cette solution de l'iodure radioactif à l'état sec ou une solution aqueuse d'iodure radioactif. On opère dans un flacon scellé tel qu'un flacon pour antibiotique fermé par une membrane de caoutchouc.

De préférence, l'iodure radioactif à l'état sec ou la solution aqueuse d'iodure radioactif a un pH d'environ 7. Lorsqu'on utilise une solution aqueuse d'iodure radioactif, cette solution présente de préférence une activité volumique d'au moins 20 millicuries par ml.

Lorsqu'on utilise de l'iodure radioactif à l'état sec, celui-ci présente de préférence une activité telle que le rapport de son activité en millicurie au poids en milligramme de l'acide gras bromé ou iodé soit au plus égal à 0,6.

A titre d'exemple de cétones susceptibles d'être utilisées, on peut citer l'acétone ou la méthyl éthyl cétone.

Le procédé, tel que caractérisé ci-dessus, permet de réduire la concentration des formes oxydées de l'iode radioactif dans la solution de marquage (iode moléculaire et ions iodates) qui, par réaction sur le substrat ou le solvant, peuvent conduire à la formation d'impuretés radioactives d'une part et diminuer le rendement de marquage d'autre part.

En effet, bien que dans les réactions d'échanges, il soit généralement préférable d'éviter la présence d'un iodure non radioactif qui participe à la réaction d'échange et diminue de ce fait le rendement de

marquage, on a trouvé que dans le procédé de la présente invention, on pouvait tirer profit de la présence d'un tel iodure entraîneur pour améliorer le rendement de marquage. Toutefois, la concentration des ions iodures froids doit être très élevée devant celle des ions iodures radioactifs et négligeable devant celle de l'acide gras halogéné. On utilise généralement une quantité d'iodure entraîneur telle que le rapport molaire de l'acide gras iodé ou bromé à l'iodure entraîneur soit de $2 \cdot 10^2$ à $10^6$.

Les ions iodures non radioactifs jouent deux rôles essentiels :

Dans les conditions de la réaction d'échange en milieu cétonique et en présence d'eau, les traces d'oxydants (oxygène moléculaire) provoquent la formation des formes oxydées de l'iode ($I_2$, $IO_3^-$...) qui immobilise une partie de l'iode radioactif. Par contre, si dans le milieu réactionnel la concentration des ions iodures radioactifs est très inférieure à celle des ions iodures froids, la fraction molaire d'iode radioactif oxydé sera négligeable, ce qui conduit à une amélioration du rendement de marquage.

Bien que la réaction soit réalisée en phase organique, la présence de traces d'eau permet d'envisager l'équilibre de dismutation :

$$3\ I_2 + 9\ H_2O \underset{1}{\overset{2}{\rightleftarrows}} IO_3^- + 5\ I^- + 6\ H_3O^-$$

par ailleurs les ions iodures sont en équilibre avec les ions $I_3^-$ :

$$I_3^- \underset{1}{\overset{2}{\rightleftarrows}} I_2 + I^-$$

L'addition d'iodures entraîneurs déplace ces équilibres dans le sens 2, ce qui diminue les concentrations des formes $IO_3^-$ et $I_2$ dans la solution.

De même, on a pu constater qu'en opérant avec de l'iodure à l'état sec à pH 7 ou avec une solution d'iodure à pH 7, plutôt qu'en milieu basique, on améliore de façon notable le rendement de marquage. Cet effet dû à la diminution du pH est certainement lié à un déplacement de l'équilibre de dismutation de l'iode dans le sens 2 qui provoque une diminution de la concentration des ions iodates.

Par ailleurs, on peut noter que le procédé de l'invention permet d'atteindre un rendement de marquage d'au moins 95 %, avec des durées d'échange relativement courtes qui généralement n'excèdent pas 5 à 7 minutes.

Après cette réaction d'échange, on évapore le solvant en continuant de chauffer après avoir introduit dans le flacon scellé une aiguille de mise à l'air. Ensuite, on traite le produit sec ainsi obtenu en vue de le mettre sous une forme injectable dans le corps humain. Dans ce but, on lui ajoute une solution tampon injectable légèrement alcaline, ayant par exemple un pH d'environ 9, telle qu'une solution tampon de carbonate de sodium ; on obtient ainsi une suspension colloïdale de l'acide gras marqué que l'on dissout dans un liquide compatible avec le corps humain, par exemple une solution de sérum-albumine humaine ou le sérum sanguin du malade à examiner.

Le procédé de marquage de l'invention conduit ainsi à l'obtention d'un produit radiopharmaceutique directement injectable sans nécessiter des opérations complexes et de longue durée. En effet, la durée de l'ensemble des opérations de marquage et de transformation du produit marqué ne solution injectable, n'excède pas 30 minutes, et ces opérations peuvent être réalisées par un personnel non spécialisé dans le laboratoire chaud d'un service hospitalier au moment de l'utilisation.

Dans ce cas, on a purifié au préalable les réactifs utilisés afin de les rendre stériles et apyrogènes. Pour faciliter les opérations, on conditionne les différents réactifs à l'exception de la solution d'iodure radioactif dans des flacons contenant chacun les quantités nécessaires pour la préparation de la dose de produit radiopharmaceutique à injecter. Ces flacons sont ensuite disposés dans une trousse afin d'avoir pour chaque opération tous les réactifs nécessaires qui ont été soumis aux contrôles voulus, et qui restent stables pendant plusieurs mois.

Ainsi, on peut limiter les frais de chaque examen scintigraphique, car les opérations de contrôle et de purification sont moins onéreuses lorsqu'elles sont effectuées sur un lot de produits conditionnés en trousse plutôt que sur chaque dose de produit marqué.

La trousse comprend un support compartimenté pour recevoir une série de flacons constitués par :

— un premier flacon contenant un acide gras cristallisé,
— un deuxième flacon contenant une cétone et un iodure entraîneur,
— un troisième flacon contenant une solution tampon injectable légèrement alcaline, et
— un quatrième flacon contenant de la sérum albumine humaine.

Selon un mode préférentiel de réalisation du procédé de l'invention, on réalise la réaction d'échange en partant du dérivé iodé de l'acide gras à marquer.

En effet, on augmente ainsi la vitesse de la réaction d'échange car l'iode est un meilleur groupe nucléophile et de ce fait, on peut obtenir une réaction complète au bout d'environ 5 minutes. De plus, le fait de partir du dérivé iodé permet d'obtenir un produit homogène, et d'éviter l'injection de dérivé bromé donc la formation in vivo de bromoacétate qui est toxique.

Les dérivés d'acides gras à longue chaîne, iodés ou bromés en oméga peuvent être obtenus par une réaction de tosylation des acides gras oméga-hydroxylés suivie d'une réaction d'échange $TsO^-$, $X^-$.

Lorsque les acides gras oméga-hydroxylés n'existent pas à l'état naturel, on peut les obtenir soit à partir des trihydroxyacides naturels par réaction de β élimination, soit par condensation d'un acide

oméga-bromé à courte chaîne sur un alcool acétylénique vrai, ce qui conduit à un oméga-hydroxyacide acétylénique que l'on réduit ensuite pour obtenir le dérivé éthylénique ou le composé saturé.

A titre d'exemple, on peut obtenir l'acide hydroxy-16 hexadécène-9 oïque E à partir de l'acide aleuritique qui existe à l'état naturel, comme cela est décrit par D. E. AMES, T. G. GOODBURN, A. W. JEVANS et J. F. Mc. GHIE J. Chem. Soc. (C), 268, 1968.

La conversion de l'acide trihydroxy-9, 10, 16 hexadécanoïque en acide hydroxy 16-hexadécène-9-oïque E ou Z est réalisée par action de l'iodure de phosphonium sur les diastéréoisomères de l'acide aleuritique suivie d'une hydrolyse alcaline.

Cependant, lorsqu'on utilise la méthode précitée, les rendements de réaction sont relativement faibles. Aussi, est-il préférable de réaliser la synthèse totale des acides gras halogénés en oméga par condensation d'un acide oméga-bromé à courte chaîne sur un alcool acétylénique. Cette méthode offre de multiples possibilités tant en ce qui concerne la longueur de la chaîne que la position de l'insaturation. Le choix de l'acide bromé en oméga et de l'alcool acétylénique permet également d'introduire plusieurs triples ou doubles liaisons dans la chaîne hydrocarbonée.

Le procédé de préparation d'un acide gras iodé répondant à la formule :

$$I(CH_2)_{n'-1}—Y—(CH_2)_{n-1}—COOH$$

dans laquelle Y représente les enchaînements $—CH_2—CH_2—$ ou $—CH=CH—$ et n et n' sont des nombres entiers de 3 à 10, se caractérise en ce que l'on condense un acide oméga bromé de formule :

$$Br—(CH_2)_{n-1}—COOH$$

et un alcool acétylénique de formule :

$$H—C \equiv C—(CH_2)_{n'}—CH_2OH,$$

dans l'ammoniac liquide en présence d'amidure de lithium pour obtenir l'acide hydroxy-oméga-alcynoïque de formule :

$$HO—(CH_2)_{n'-1}—C \equiv C—(CH_2)_{n-1}—COOH,$$

en ce que l'on soumet ensuite à une réduction l'acide hydroxy-oméga-alcynoïque pour obtenir l'acide hydroxy-oméga-alcénoïque ou l'acide hydroxy-oméga-alcanoïque correspondant, en ce que l'on fait réagir l'acide hydroxy-oméga-alcanoïque ou l'acide hydroxy-oméga-alcénoïque ainsi obtenu avec du chlorure de paratoluène sulfonyle pour obtenir le dérivé tosylé d'acide gras correspondant et en ce que l'on soumet le dérivé tosylé ainsi obtenu à une réaction d'échange tosylate-iode pour obtenir le dérivé iodé correspondant.

Selon une variante de ce procédé, on prépare un acide gras iodé répondant à la formule :

$$I(CH_2)_{n'-1}—C \equiv C—(CH_2)_{n-1}—COOH$$

dans laquelle n et n' sont des nombres entiers de 3 à 10 en soumettant directement l'acide hydroxy-ω alcynoïque aux réactions de tosylation et d'échange.

Le schéma réactionnel ci-dessous résume les différentes étapes de ces procédés de synthèse :

(Voir dessin page 5)

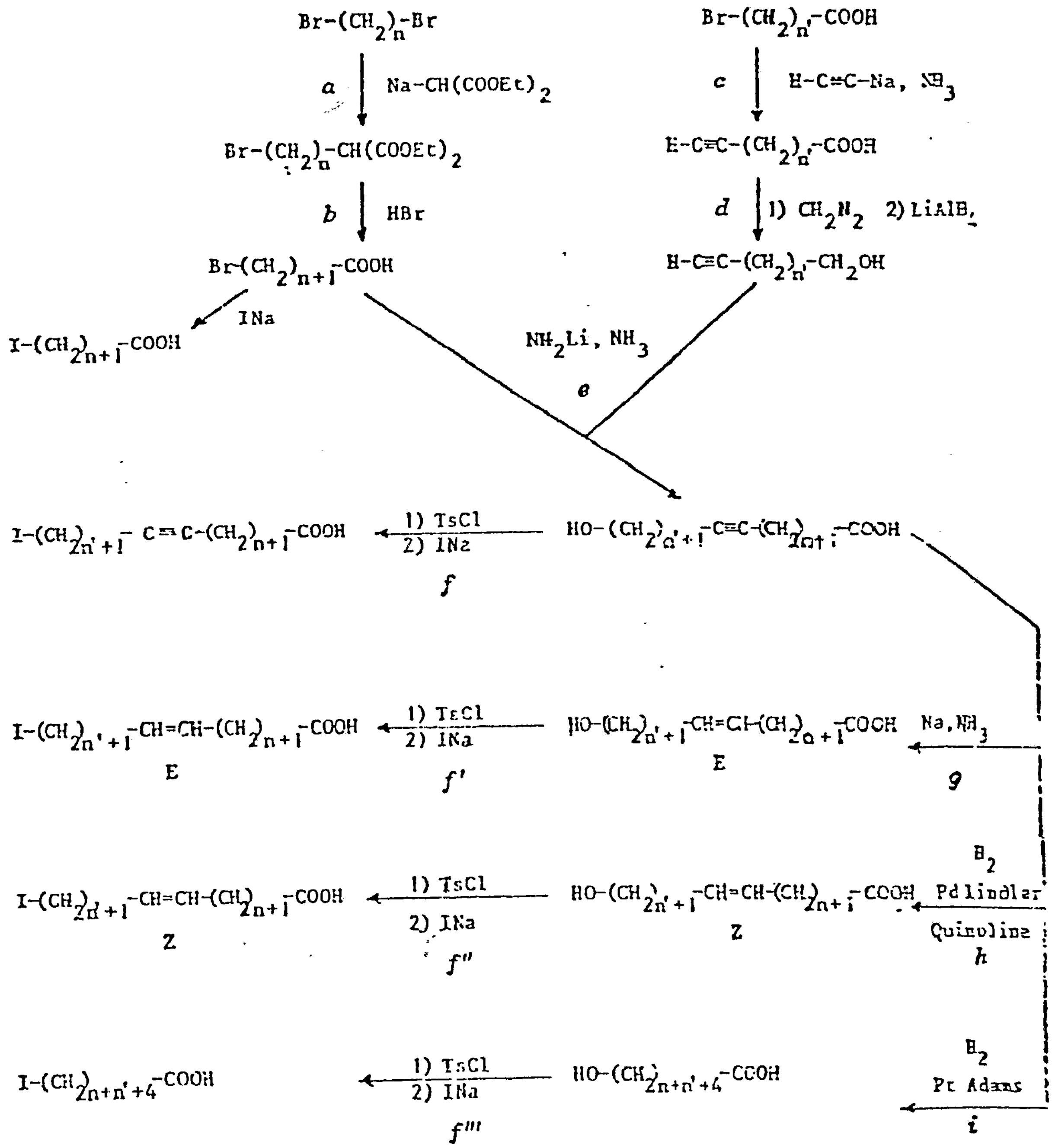


Les alcools acétyléniques vrais utilisés comme produits de départ pour cette synthèse peuvent être préparés par substitution nucléophile de l'ion acétylure sur le sel d'un acide bromo-ω alcanoïque dans l'ammoniac liquide selon le schéma réactionnel suivant :

$$Br—(CH_2)_n—COOH \xrightarrow{H—C\equiv C—Na,NH_3} H—C\equiv C—(CH_2)_{n'}—COOH$$

et par réduction du groupe carboxylique de l'acide obtenu par traitement avec le diazométhane suivie d'une réduction dans l'éther, selon le schéma réactionnel suivant :

$$H—C\equiv C—(CH_2)_n—COOH \xrightarrow[2)\ LiAlH_4]{1)\ CH_2N_2} H—C\equiv C—(CH_2)_{n'}—CH_2OH$$

Les acides bromo-ω alcanoïques utilisés comme produits de départ pour cette synthèse peuvent être obtenus par réaction du dibromo-1 ω alcane sur le dérivé sodé du malonate d'éthyle, ce qui conduit aux produits de di- et de mono-condensation, ce dernier étant isolé par distillation du mélange réactionnel sous pression réduite :

$$Br{-}(CH_2)_n{-}Br \xrightarrow{NaCH(COOEt)_2} Br{-}(CH_2)_n{-}CH(COOEt)_2 + (EtOCO)_2CH{-}(CH_2)_n{-}CH(COOEt)_2 + NaBr$$

suivie d'une décarboxylation en milieu acide (HBr) du bromo-ω alkylmalonate d'éthyle pour conduire à l'acide bromo-ω alcanoïque, selon le schéma suivant :

$$Br{-}(CH_2)_n{-}CH(COOEt)_2 \xrightarrow{HBr/H_2O} Br{-}(CH_2)_{n-1}{-}COOH$$

La réaction de condensation (e) d'un acide bromé en ω et d'un alcool acétylénique vrai pour former un acide hydroxy-ω alcynoïque est réalisée dans l'ammoniac liquide en présence d'amidure de lithium. Le choix du lithium s'explique par le fait que ce métal bloque la fonction OH : la réaction de l'acide bromé sur le dérivé dilithié de l'alcool acétylénique ne provoque pas de O-alkylation ; seule la C-alkylation est observée. L'alcool acétylénique doit être utilisé en grand excès par rapport à l'acide bromé (3 à 10 moles pour 1 Mole) car la vitesse de la réaction de condensation étant très faible à – 35 °C, on risque d'observer la duplication de l'acide bromé ou la formation d'amine.

L'acide hydroxy-ω alcynoïque obtenu est ensuite soumis à une réduction partielle ou totale pour former l'acide hydroxy-ω alcénoïque ou alcanoïque correspondant qui pourra être transformé ensuite par tosylation et iodation du groupe hydroxyle terminal, en acide iodé saturé ou éthylénique Z ou E :

— pour obtenir les acides iodo-ω alcanoïques, on réalise une hydrogénation totale des acides hydroxy-ω alcynoïques. Le catalyseur employé est le platine d'Adams ($PtO_2$) et la réaction est effectuée soit dans l'acide acétique, soit dans un alcool tel que le propanol, soit dans l'éther,

— pour obtenir les acides iodo-ω alcénoïques Z, on réalise une hydrogénation catalytique partielle et hautement stéréosélective des acides hydroxy-ω alcynoïque au moyen du catalyseur de Lindlar (catalyseur à 5 % de palladium sur carbonate de calcium désactivé à 95 °C par une solution d'acétate de plomb) en présence de quinoline.

Pour obtenir les acides iodo-ω alcénoïques E, on réalise une réduction stéréospécifique des acides hydroxy-ω alcynoïques par voie chimique ; le réducteur utilisé est le sodium en solution dans l'ammoniac liquide sous pression à une température de 20-60 °C.

Les acides gras hydroxylés en position ω sont ensuite soumis aux réactions de tosylation et d'échange (f, f', f" ou f"') pour obtenir les acides gras iodés en position ω.

La réaction de tosylation s'effectue dans la pyridine ou dans un solvant tel que le dichlorométhane. Afin d'éviter l'élimination, la réaction est réalisée dans tous les cas à basse température : entre 0 et 5 °C. Le temps de réaction varie de 2 h 30 à 24 heures lorsque l'on utilise la pyridine comme solvant, tandis qu'il est de huit jours si l'on emploie le dichlorométhane.

On réalise donc de préférence cette réaction dans la pyridine, en utilisant un léger excès de chlorure de para-toluènesulfonyle par rapport à l'alcool.

La réaction d'échange est une attaque du tosylate, très bon groupe partant, par l'ion nucléophile $I^-$.

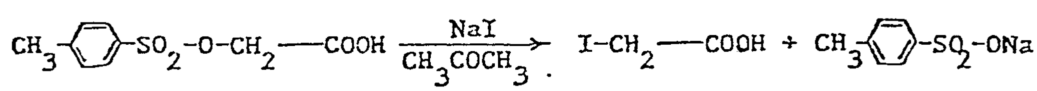


Cette réaction d'échange tosylate-iode est comparable à l'échange brome-iode, elle se déroule dans l'acétone, au reflux selon la méthode de GENSLER et THOMAS et elle doit être conduite sous atmosphère d'azote.

Avant d'être mis en œuvre dans le procédé de l'invention, les dérivés iodés ou bromés sont purifiés par chromatographie liquide à haute performance en utilisant un support à base de silice et un éluant constitué essentiellement par de l'heptane, de l'éther et de l'acide acétique. Généralement pour cette étape de purification, on utilise un chromatographe HPLC équipé d'un réfractomètre différentiel et d'une ou deux colonnes de silice du type Prep Pak 500 et en réalisant l'élution, au moyen d'un mélange de 97,8 % d'heptane, 1,8 % d'éther et 0,4 % d'acide acétique avec un débit de 200 ml par min., sous une pression de 5 bars, en injection un échantillon de 3 à 5 g.

On peut augmenter le rendement de purification en montant en série deux colonnes de silice et en augmentant la polarité du solvant constitué dans ce cas par un mélange de 95,6 % d'heptane, 4 % d'éther et 0,4 % d'acide acétique avec un débit de 50 ml par min. et une pression de 8 bars. Après cette étape de purification, le dérivé iodé ou bromé est soumis à une stérilisation puis conditionné dans des flacons.

D'autres avantages et caractéristiques de l'invention apparaîtront mieux à la lecture des exemples suivants donnés bien entendu à titre illustratif et non limitatif.

Exemple 1 : Préparation d'acides gras iodés en position ω.

1) Préparation des alcools acétyléniques

a) Préparation de l'acide alcyne-oïque

$$Br{-}(CH_2)_n{-}COOH \xrightarrow[NH_3]{H{-}C\equiv C{-}Na} HOCO{-}(CH_2)_{n'}{-}C\ C{-}H$$

Dans un tricol contenant 2 l d'ammoniac liquide et sous courant d'acétylène, on dissout 40 g de Na en maintenant l'agitation. L'addition d'acétylène est poursuivie jusqu'à disparition totale de la couleur bleue du sodium en solution. On ajoute lentement 0,49 mole d'acide bromo-ω alcanoïque en solution dans 150 ml de THF, puis 150 ml de THF pur. Le mélange est laissé sous agitation pendant 15 heures jusqu'à évaporation totale de l'ammoniac. Le précipité est récupéré avec un peu d'éther et hydrolysé. Le mélange réactionnel est acidifié (HCl 11N) et le produit est extrait à l'éther. La phase éthérée est lavée à l'eau, filtrée sur papier Whatman séparateur de phases, et évaporée. Le produit est distillé sous pression réduite :

Acide octyne-7 oïque (n'= 5) : $H{-}C\ C{-}(CH_2)_5{-}COOH$

$Eb_{0,6}$ = 95-100 °C, rendement 65 %

Acide nonyne-8 oïque (n' = 6) : $H{-}C\ C{-}(CH_2)_6{-}COOH$

$Eb_{0,5}$ = 106-108 °C ; rendement 65 %

b) Transformation de l'acide en alcool acétylénique

Le diazométhane en solution dans l'éther est ajouté au composé obtenu en a) jusqu'à persistance de la coloration jaune. L'excès de diazométhane est détruit par quelques gouttes d'acide acétique. Le volume d'éther est ramené à 200 ml par évaporation. On obtient alors 0,3 mole d'ester, le rendement de l'estérification est en effet pratiquement quantitatif.

$$EtOCO{-}(CH_2)_{n'}{-}C\equiv C{-}H \xrightarrow{LiAlH_4} HOCH_2{-}(CH_2)_{n'}{-}C\equiv C{-}H$$

Dans un ballon à trois cols de 2 l, adapté d'un réfrigérant, d'une ampoule à brome et d'un agitateur, on met en suspension 11,4 g de $LiAlH_4$ dans 500 ml d'éther anhydre, le mélange étant maintenu à 0 °C. L'ester en solution dans l'éther (0,3 mole dans 200 ml) est ajouté goutte à goutte et l'agitation est poursuivie 2 heures (0 °C). La solution est alors hydrolysée avec 80 ml de NaOH 5N (jusqu'à floculation). La phase solide est lavée à l'éther. La phase éthérée est lavée avec une solution de chlorure d'ammonium saturée, filtrée sur papier Whatman séparateur de phase ; l'éther est évaporé et le résidu séché parè distillation azéotropique (benzène) et distillé sous pression réduite :

Octyne-7 ol 1 : $Eb_{0,6}$ = 74 °C ; rendement 65 %

Nonyne-8 ol 1 : $Eb_{0,6}$ = 85 °C ; rendement 90 %

2) Préparation de l'acide ω-bromé

a) Préparation du bromo-ω alkylmalonate d'éthyle

$$Br{-}(CH_2)_n{-}Br + NaCH(COOEt)_2 \longrightarrow Br{-}(CH_2)_n{-}CH(COOEt)_2 + NaBr$$

Dans un tricol muni d'un réfrigérant, d'une ampoule à brome et d'un agitateur, 0,45 mole (10,35 g) de Na sont dissous dans 140 ml d'éthanol absolu distillé sur magnésium. A la solution refroidie (20 °C), on ajoute lentement 0,90 mole (133,2 g) de malonate d'éthyle distillé ($Eb_{15}$ = 102 °C). Le mélange est chauffé pendant une heure à 60 °C au bain-marie puis refroidi à 0 °C. On ajoute alors lentement 0,49 mole de dibromo 1-ω alcane dissous dans 98 ml d'éther anhydre. Le mélange réactionnel est laissé 3 jours à température ambiante. L'éther et l'éthanol sont évaporés, le résidu repris par 200 ml d'eau est extrait 4 fois avec 250 ml d'éther. La phase éthérée est lavée à l'eau, filtrée sur papier Whatman séparateur de phases et séchée sur $Na_2SO_4$. L'éther est évaporé et les produits réactionnels distillés sous pression réduite. On recueille les fractions suivantes :

(Bromo-5 pentyl) malonate d'éthyle :

$Br{-}(CH_2)_5{-}CH(COOEt)_2$

1 : Malonate d'éthyle $CH_2(COOEt)_2$ : $Eb_1$ = 64 °C

2 : Dibromopentane $Br{-}(CH_2)_5{-}Br$ : $Eb_1$ = 74 °C

3 : (Bromo-5 pentyl) malonate d'éthyle

$Br{-}(CH_2)_5{-}CH(COOEt)_2$ : $Eb_1$ = 154 °C ; rendement 43 %

4 : Tétraester $(EtOCO)_2CH{-}(CH_2)_5{-}CH(COOEt)_2$ : $Eb_1 > 160$ °C.

(Bromo-6 hexyl) malonate d'éthyle :

$Br{-}(CH_2)_6{-}CH(COOEt)_2$

1 : Malonate d'éthyle : $Eb_{13}$ = 95-100 °C

2 : Dibromohexane $Br{-}(CH_2)_6{-}Br$ : $Eb_{13}$ = 90 °C

3 : (Bromo-6 hexyl) malonate d'éthyle $Br{-}(CH_2)_6{-}CH(COOEt)_2$ : $Eb_{0,3}$ = 139 °C, $n_D^{20}$ = 1,460, rendement 41 %

4 : Tétraester de l'acide dicarboxysébacique $(EtOCO)_2{-}CH{-}(CH_2)_6{-}CH(COOEt)_2$ : $Eb_{0,3} > 139$ °C.

(Bromo-10 décyl) malonate d'éthyle :

$Br-(CH_2)_{10}-CH(COOEt)_2$
1 : Malonate d'éthyle : $Eb_1$ = 64 °C
2 : Dibromodécane $Br-(CH_2)_{10}-Br$ : $Eb_1$ = 150 °C
3 : (Bromo-10 décyl) malonate d'éthyle $Br-(CH_2)_{10}-CH(COOEt)_2$ : $Eb_1$ = 192 °C. Rendement 61 %
4 : Tétraester $(EtOCO)_2CH-(CH_2)_{10}-CH(COOEt)_2$ : $Eb_1$ = 195 °C

b) Transformation en acide bromo-ω alcanoïque

$$Br-(CH_2)_n-CH(COOEt)_2 \xrightarrow{HBr} Br-(CH_2)_{n-1}-COOH$$

On chauffe 0,18 Mole de dérivé malonique et 51 g d'HBr à 48 % dans l'eau dans un tricol muni d'une ampoule à brome et d'une colonne à distiller équipée d'un réfrigérant descendant. Le mélange réactionnel est porté à 120-150 °C. Le bromure d'éthyle distille en tête avec l'eau et HBr. Au fur et à mesure que la distillation se poursuit, on rajoute dans le tricol une quantité d'acide bromhydrique égale à celle du distillat recueilli (150 ml au total). Le mélange est chauffé 4 heures. Après refroidissement, le produit est hydrolysé à l'aide de glace et de 250 ml d'eau.

L'acide précipite à l'état solide. Il est extrait à l'éther (500 ml). La phase éthérée est lavée une fois à l'eau, et évaporée. Enfin, l'acide brut est séché par distillation azéotropique (ØH) et distillé sous pression réduite :

Acide bromo-7 heptanoïque $Br-(CH_2)_6-COOH$ : $Eb_{0,8}$ = 124 °C ; rendement 74 %
Acide bromo-8 octanoïque $Br-(CH_2)_7-COOH$ : $Eb_{0,8}$ = 142 °C ; rendement 67,5 %
Acide bromo-12 dodécanoïque $Br-(CH_2)_{11}-COOH$ : $Eb_{0,8}$ = 174 °C ; rendement 30,5 %

3) Préparation de l'acide hydroxy-ω alcynoïque

La réaction est réalisée dans un tricol équipé d'une agitation mécanique, d'une ampoule à brome et d'un réfrigérant à carboglace, acétone (– 80 °C). A 0,32 mole d'amidure de lithium (obtenue par réaction de 2,24 g de Li en présence de nitrate ferrique) en solution dans 1,5 l d'ammoniac liquide, on ajoute 0,124 mole d'alcool acétylénique en solution dans 100 ml de THF anhydre (en 30 mm). L'agitation est poursuivie 45 mn après la fin de l'addition. On ajoute goutte à goutte 0,048 mole d'acide bromo-alcanoïque en solution dans 200 ml de THF anhydre. Le mélange réactionnel est maintenu sous agitation pendant 20 heures. Après évaporation de l'ammoniac, le mélange est hydrolysé par addition de glace. Le pH de la solution est amené à 3 (HCl 11N) et l'acide est extrait à l'éther, puis séché par distillation azéotropique (benzène). Il est recristallisé dans un mélange éther-pentane.

| | Rendement | F°C | Analyse élémentaire | | | |
|---|---|---|---|---|---|---|
| | | | C | | H | |
| | | | calculé | obtenu | calculé | obtenu |
| Acide hydroxy-14 tétradécyne-7 oïque (n =4 , n' =5 ) | 60 % | 38-41 | 70,0 | 69,85 | 10,0 | 10,11 |
| Acide hydroxy-16 hexadécyne-9 oïque (n =6 , n' =5 ) | 76 % | 60-62 | 71,64 | 71,74 | 10,45 | 10,40 |
| Acide hydroxy-20 éicosyne-12 oïque (n =9 , n' =6 ) | 63,5 % | 77-79 | 74,07 | 73,46 | 11,11 | 11,18 |

4) Hydrogénation catalytique partielle en acide hydroxy-ω alcénoïque Z

L'hydrogénation est réalisée dans un appareil comprenant une réserve d'hydrogène, une enceinte d'hydrogénation et un dispositif de vide. Dans un erlen contenant 40 ml de propanol ou d'éther sont dissous $3,7 \cdot 10^{-3}$ mole d'acide hydroxy-ω alcynoïque et 450 mg de quinoline.

On ajoute alors 150 mg de catalyseur de Lindlar et l'erlen est fixé à l'enceinte d'hydrogénation. Après purge de l'enceinte, l'agitation est mise en route et la consommation d'hydrogène mesurée en fonction du temps, à pression ordinaire. L'hydrogénation terminée, la solution est filtrée, le propanol évaporé, et le résidu repris avec 100 ml d'éther qui sont lavés deux fois à l'eau acidulée (HCl) et deux fois à l'eau. La phase éthérée est filtrée sur papier Whatman séparateur de phases puis évaporée. Le produit est analysé.

Les deux acides éthyléniques Z synthétisés (en $C_{14}$ et $C_{16}$) sont liquides à température ordinaire. Les rendements sont de l'ordre de 80 %.

5) Hydrogénation totale en acide hydroxy-ω alcanoïque

Le mode opératoire est identique au précédent. La réaction est réalisée sur $3,7 \cdot 10^{-3}$ mole d'acide hydroxy-ω alcynoïque en présence de 20 mg de $PtO_2$. Les produits sont recristallisés dans l'acétone. Les rendements sont de 70 à 80 %.

6) Hydrogénation partielle en acide hydroxy-ω alcénoïque E

L'acide hydroxy-ω alcynoïque ($3,7 \cdot 10^{-3}$ mole) est dissous dans 50 ml d'éther anhydre et versé dans un autoclave de 500 ml. L'ensemble est refroidi à − 50 °C (acétone, carboglace). On ajoute alors rapidement 2 g de sodium dissous dans 150 ml d'ammoniac liquide. L'autoclave est fermé et agité pendant un jour dans un bain-marie à 57 °C. L'ammoniac restant est évaporé et le mélange réactionnel est hydrolysé (glace + eau). Le milieu est acidifié (HCl 11N) et le produit extrait à l'éther. La phase éthérée est lavée à l'eau, filtrée sur papier Whatman séparateur de phases, évaporée. Le produit est analysé. Rendement 50 à 60 %.

7) Tosylation de l'ω-hydroxyacide

$3,6 \cdot 10^{-3}$ mole d'ω-hydroxyacide sont dissous dans 6 ml de pyridine. La solution est maintenue sous agitation à 0 °C. 0,86 g ($4,5 \cdot 10^{-3}$ mole) de chlorure-de paratoluène sulfonyle sont ajoutés par portions. L'agitation est poursuivie pendant 8 heures, puis l'ensemble est maintenu à 5 °C pendant 10 à 48 heures. La réaction est suivie par chromatographie sur couche mince (éluant : éther, pentane 7/3, support $SiO_2$, révélation par chauffage de la plaque après pulvérisation avec une solution de chlorure de cobalt à 1 % dans $H_2SO_4$ à 10 %). Le milieu réactionnel est hydrolysé par addition lente de 1 ml d'eau puis addition plus rapide de 6 ml d'eau. Le tosylate de l'acide gras est alors extrait par $CH_2Cl_2$, et recristallisé dans le pentane à − 20 °C.

8) Iodation du tosylate d'acide gras

La réaction d'iodation du tosylate d'acide gras est conduite sous atmosphère d'azote. Le tosylate précédent est dissous dans 20 ml d'acétone et 2,2 g ($14,8 \cdot 10^{-3}$ mole) de NaI sont ajoutés. Le mélange réactionnel est porté au reflux pendant deux heures. La solution est filtrée, l'acétone évaporée, et le produit repris dans 100 ml d'éther. La phase éthérée est lavée deux fois avec 50 ml d'eau additionnée de métabisulfite de sodium ($Na_2S_2O_5$, 20 %) puis évaporée.

Le produit est séché par distillation azéotropique (acétone) puis recristallisé dans le pentane à − 20 °C. Il est alors analysé :

Acide iodo-14 tétradécène-7 oïque Z :
F < 20 °C
Acide iodo-14 tétradécanoïque :
F = 65-67 °C

On a obtenu de la même façon les acides gras iodés du tableau qui suit :

(Voir Tableau page 10)

| N° | | F°C | Rdt par rapport au dérivé hydroxylé | Analyse élémentaire | | | | | |
|---|---|---|---|---|---|---|---|---|---|
| | | | | C | | H | | I | |
| | | | | calculé | obtenu | calculé | obtenu | calculé | obtenu |
| 1 | Acide iodo-16 hexadécyne-9 oïque | 35-37 | 29 | 50,79 | 51,07 | 7,14 | 7,30 | 33,59 | 31,18 |
| 2 | Acide iodo-20 eicosyne-12 oïque | 46-50 | 22 | 55,29 | 57,71 | 8,06 | 8,46 | – | – |
| 3 | Acide iodo-16 hexadécène-9 oïque E | 49-51 | 39 | 50,53 | 50,20 | 7,63 | 7,80 | 33,42 | 32,42 |
| 4 | Acide iodo-16 hexadécanoïque | 65-67 | 20 | 50,26 | 51,46 | 8,11 | 8,22 | 33,24 | 30,96 |
| 5 | Acide iodo-20 eicosanoïque | 79-80 | 25 | 54,79 | 54,85 | 8,88 | 9,08 | 28,99 | 26,34 |

Exemple 2

Cet exemple illustre la préparation d'acide iodo-16 hexadécène-9 oïque E au moyen d'iode 123.

Dans cet exemple, on ajoute dans un flacon contenant une certaine quantité d'acide gras cristallisé 1,4 ml d'acétone contenant 3,5 µg d'iodure de sodium, puis on ajoute le contenu de ce flacon dans un autre flacon contenant 35 microlitres d'iodure de sodium 123, ce qui représente une activité de 1 millicurie ; puis on chauffe le flacon dans un bain-marie sec à 103 °C pendant 5 minutes.

A la fin de cette opération, on détermine le rendement de marquage par électrophorèse en milieu Véronal à 200 volts pendant 30 minutes pour faire migrer les ions iodure et par comptage de pics de radioactivité sur les différentes zones du papier d'électrophorèse. Les résultats obtenus lorsqu'on réalise le marquage avec différentes quantités d'acide gras sont donnés dans le tableau ci-après.

| Acide gras iodé de départ (en mg) | Quantité d'INa par mg d'acide gras | Quantité I 123 par mg d'acide gras | Rendement |
|---|---|---|---|
| 2 mg | 1,75 µg | 500 µCuries | 92,1 % |
| 3 mg | 1,17 µg | 333 µCuries | 91,9 % |
| 3,5 mg | 1 µg | 285 µCuries | 95,8 % |

Au vu de ce tableau, on constate que l'on obtient de très bons résultats avec 285 millicuries d'iode radioactif par gramme d'acide gras.

Exemple 3

Cet exemple illustre l'influence du pH de la solution d'iodure radioactif sur les résultats obtenus.

On dissout 2 mg d'acide iodo-16 hexadécène-9 oïque dans 2 ml d'acétone contenant 5 microgrammes d'iodure de sodium, et on ajoute la solution obtenue à 40 microlitres de solution d'iodure de sodium radioactif ayant une activité totale de 1 millicurie, on chauffe ensuite l'ensemble à 103 °C dans un bain-marie sec pendant 5 minutes.

A la fin de cette opération, on détermine le rendement de marquage par électrophorèse et par comptage comme précédemment. Lorsqu'on utilise une solution d'iodure à pH de 12,3, le rendement de marquage est de 85,2 % alors qu'en utilisant une solution d'iodure à pH sensiblement égal à 7, le rendement de marquage est de 93,3 %. Ainsi, on constate que l'utilisation d'une solution d'iodure à pH 7 permet d'augmenter de façon notable le rendement de marquage.

Exemple 4

Cet exemple illustre l'influence de l'activité volumique de la solution aqueuse d'iodure radioactif et des volumes de solution d'iodure et de solution d'acide gras utilisés, sur les résultats obtenus.

Dans cet exemple, on utilise pour le marquage une solution comprenant 2,5 mg/ml d'acide iodo-16 hexadécène-9 oïque dans de l'acétone contenant de l'iodure de sodium entraîneur avec un rapport molaire acide gras/iodure entraîneur égal à $4 \cdot 10^2$. On ajoute à la solution d'acétone contenant l'acide gras et l'iodure entraîneur une certaine quantité de solution aqueuse d'iodure de sodium radioactif, puis on porte l'ensemble à une température de 102-104 °C dans un bain-marie sec pendant 5 min. A la fin de cette opération, on détermine le rendement de marquage par chromatographie sur résine anionique DOWEX AG-1 X8 forme $Cl^-$ : la résine est conditionnée sous acétone, une aliquote de la solution de marquage placée sur la résine est éluée par ce solvant, les iodures sont retenus, l'acide gras radioactif est élué ;

$$\text{Rendement de marquage} = \frac{\text{activité de l'éluat}}{\text{activité éluat} + \text{activité résine}}$$

Les résultats obtenus pour des volumes différents de solution d'acide gras dans l'acétone et de solution d'iodure de sodium radioactif sont donnés dans le tableau I ci-joint.

Ces résultats sont confirmés par analyse CCM (support cellulose, éluant : heptane : 380 V, éther : 80 V, Acide acétique I V). Le Rf de l'acide gras est compris entre 0,9 et 1, celui des espèces ioniques est peu différent de 0. Les bandes de cellulose correspondant à des Rf (0-0,2), ... (0,8-1) sont récupérées, leur radioactivité est mesurée.

$$\text{Rendement de marquage} = \frac{\text{Radioactivité Rf (0,8 à 1)}}{\text{Radioactivité totale.}}$$

La qualité du produit marqué est contrôlée par HPLC analytique en utilisant les conditions qui suivent :

— une phase stationnaire, $SiO_2$ (10 μm)

— Eluant : heptane 98,1 %
éther 1,5 %
Acide acétique 0,4 %

— débit : 7 ml/minute

La colonne est préalablement conditionnée à l'aide d'une solution de n-heptane contenant 3 % d'acide acétique.

La solution brute de marquage est analysée selon la méthode qui suit : l'acétone de la solution de marquage est évaporée (sans perte de radioactivité) ; l'acide gras marqué, analysé en solution dans l'éluant, conduit à un seul produit radioactif qui absorbe en UV (≃ 7,5 volumes de colonne). Etudié en RMN $^1H$, ce produit présente les caractéristiques de l'acide gras iodé initial. Dans tous les cas étudiés, l'analyse HPLC ne révèle pas d'impuretés ($>$1 %) radioactives ou absorbant en UV.

Notons par ailleurs qu'il n'est pas possible d'analyser directement la solution de marquage dans l'acétone pour les raisons qui suivent :

— l'acétone contient souvent des impuretés qui absorbent en UV,

— il semble que l'acide gras et ce solvant conduisent à des associations séparées en HPLC.

Au vu du tableau I, on constate que les meilleurs résultats sont obtenus lorsqu'on utilise un volume de solution d'iodure de sodium de 80 microlitres représentant une activité totale de 2 millicuries et un volume de solution d'acide gras de 2,8 millilitres ayant une concentration en acide gras de 2,5 mg/ml et une concentration en iodure entraîneur telle que le rapport molaire acide gras/NaI entraîneur soit de $4 \cdot 10^2$.

Par ailleurs, on a constaté que des durées de chauffage supérieurs à 5 min. n'avaient pas d'effet sur le rendement de marquage.

Enfin, on peut déduire de ces résultats que le rapport volume de solution d'acide gras/volume de solution de $Na^{123}I$ doit être d'au moins 14.

## Exemple 5

Dans cet exemple, on étudie l'influence de rapport entre le volume de solution aqueuse et le volume de solution d'acide gras sur le rendement de marquage, dans le cas du marquage à l'iode 131 de l'acide iodo-16 hexadécène-9 oïque.

Dans ce cas, on utilise le même mode opératoire que dans l'exemple 4. Les résultats obtenus sont donnés dans le tableau II annexé, qui mentionne également les conditions utilisées.

## Exemple 6

Cet exemple illustre la préparation d'une solution injectable d'acide gras marqué à l'iode 123 par le procédé de l'invention.

Chacun des constituants nécessaires pour le marquage et pour l'obtention d'une solution injectable est conditionné de façon stérile et apyrogène dans un flacon type « pénicilline ». Ces flacons qui ont été préparés au préalable et soumis à tous les contrôles nécessaires pour leur utilisation en médecine, sont disposés dans une trousse qui comprend ainsi :

— un flacon A contenant 7 mg d'acide iodo-16 hexadécène-9 oïque cristallisé,

— un flacon B contenant de l'acétone et de l'iodure de sodium,

— un flacon C contenant 5 ml de solution tampon à pH 9 ayant la composition suivante :
0,28 mg de carbonate de sodium anhydre, et
1,6 mg de bicarbonate de sodium par ml de solution de chlorure de sodium à 9 pour 1 000.

— un flacon D contenant de l'albumine humaine en solution à 20 % dans du sérum physiologique.

Pour le marquage, on utilise de plus un flacon contenant la quantité nécessaire de solution aqueuse d'iodure radioactif qui est livré au dernier moment. Ce flacon contient 40 microlitres de solution aqueuse d'iodure de sodium à pH 7 ayant une activité totale en iode 123 de 1 millicurie.

Pour réaliser le marquage, on prélève dans le flacon B 2,8 $cm^3$ d'acétone contenant 7 μg d'iodure de sodium et on l'ajoute au contenu du flacon A, c'est-à-dire aux 7 mg d'acide gras cristallisé, ce qui permet d'obtenir une solution de l'acide gras dans l'acétone contenant la quantité voulue d'iodure de sodium entraîneur.

On prélève alors 1,4 $cm^3$ de la solution du flacon A et on l'ajoute dans le flacon scellé par une membrane de caoutchouc qui contient la solution d'iodure radioactif à pH 7. On place ensuite ce flacon scellé contenant la solution d'iodure radioactif et la solution d'acide gras dans un bain-marie sec et on le chauffe à une température de 102-103 °C pendant 5 minutes pour réaliser la réaction d'échange et

obtenir l'acide gras marqué par $^{123}I$. Après ce chauffage, on introduit une aiguille de mise à l'air dans la membrane en caoutchouc qui ferme le flacon afin d'éliminer l'acétone par évaporation. Après cette opération, on prélève dans le flacon C 3 ml de la solution tampon et on l'introduit dans le flacon contenant l'acide gras marqué, puis on place celui-ci dans un bain-marie sec en soumettant le contenu à une agitation et en chauffant pendant environ 15 minutes à 70 °C pour obtenir une suspension colloïdale d'acide gras marqué. Lorsque la suspension est un peu refroidie, on ajoute alors 1 ml de sérum albumine humaine que l'on prélève dans le flacon D, la solution devient alors limpide : la solution d'acide gras iodé marqué par l'iode 123 ainsi obtenue est injectée directement par voie intraveineuse.

Exemple 7

Cet exemple illustre la préparation d'une solution injectable d'acide gras marqué à l'iode 123 par le procédé de l'invention, mais en utilisant de l'iodure radioactif à l'état sec au lieu d'une solution aqueuse d'iodure radioactif.

Cet iodure radioactif à l'état sec a été obtenu par évaporation d'une solution aqueuse d'iodure 123 radioactif produit en cyclotron et livré à pH 7 avec une activité de 10 millicuries par millilitre. Pour transformer cette solution en iodure radioactif sec, on introduit dans un flacon type pénicilline de livraison, un volume égal à l'activité désirée et calibrée pour l'heure d'utilisation. On porte ensuite le flacon dans un bain thermostaté sec à 100 °C sans bouchon et on évapore à sec. On bouche ensuite le flacon, on le sertit et on le soumet à une stérilisation à l'autoclave pendant 20 minutes à 120 °C.

Les constituants nécessaires pour le marquage sont dans une trousse comprenant comme dans l'exemple 6,

— un flacon A contenant 7 mg d'acide iodo-16 hexadécène-9 oïque cristallisé,
— un flacon B contenant de l'acétone et de l'iodure de sodium,
— un flacon C contenant 5 ml de la solution tampon à pH 9, et
— un flacon D contenant de l'albumine humaine en solution à 20 % dans du sérum physiologique.

Pour le marquage, on utilise de plus, un flacon contenant l'activité nécessaire en iodure radioactif à l'état sec qui est livré au dernier moment. Ce flacon contient 4 millicuries d'iodure radioactif à pH 7 à l'état sec.

Pour réaliser le marquage on prélève dans le flacon B 1 ml d'acétone contenant 2,5 µg d'iodure de sodium et on l'ajoute au contenu du flacon A, c'est-à-dire aux 7 mg d'acide gras cristallisé, ce qui permet d'obtenir une solution d'acide gras dans l'acétone contenant la quantité voulue d'iodure de sodium entraîneur, on prélève alors la totalité de la solution du flacon A (1 ml) et on l'ajoute dans le flacon scellé par une membrane en teflon qui contient l'iodure radioactif à l'état sec à pH 7. On place ensuite ce flacon scellé contenant l'iodure radioactif et la solution d'acides gras dans un bain-marie sec et on le chauffe à une température de 102-103 °C pendant 5 minutes pour réaliser la réaction d'échange et obtenir l'acide gras marqué par l'iode 123. Après ce chauffage, on introduit une aiguille de mise à l'air dans la membrane en téflon qui ferme le flacon afin d'éliminer l'acétone par évaporation. Après cette opération, on prélève dans le flacon C 4 ml de la solution tampon et on l'introduit dans le flacon contenant l'acide gras marqué, puis on place celui-ci dans un bain-marie sec en soumettant le contenu à une agitation et en chauffant pendant environ 15 minutes à 70 °C pour obtenir une suspension d'acide gras marqué. Lorsque la suspension est refroidie on ajoute alors 1,5 ml de sérum albumine humaine que l'on prélève dans le flacon D, la solution devient alors limpide.

Avant de préparer la solution injectable, on a déterminé le rendement de marquage et l'on obtient un rendement de marquage de 98,3 %, ce qui correspond à 1,7 % d'iodure libre radioactif.

On remarque ainsi que le fait d'opérer avec de l'iodure radioactif à l'état sec permet d'obtenir un meilleur résultat.

Pour vérifier ce fait, on effectue différents marquages sur l'acide iodo-16 hexadécène-9 oïque dans les mêmes conditions que précédemment, mais en utilisant des solutions aqueuses d'iodure radioactif présentant chacune une activité totale de 4 millicuries mais ayant des volumes différents (30, 200 et 500 µl). On détermine également le rendement de marquage. Les résultats obtenus sont donnés dans le tableau qui suit.

| iodure radioactif (4 millicuries) | à l'état sec | dans 30 µl le solution | dans 200 µl de solution | dans 500 µl de solution |
|---|---|---|---|---|
| Rendement de marquage | 98,3 % | 93,8 % | 85,9 % | 59,8 % |

Tableau I

| Solution de $Na^{123}I$ | | Solution d'acide gras dans l'acétone + NaI entraîneur | | | Chauffage | | Rendement de marquage |
|---|---|---|---|---|---|---|---|
| Volume (µl) | Activité totale (mCi) | Volume (ml) | Concentration en acide gras (mg/ml) | Rapport molaire acide gras/NaI | T(°C) | Durée (min) | |
| 80 | 2 | 2,8 | 2,5 | $4.10^2$ | 102-104 | 5 | 96,7 |
| 144 | 2 | 4 | " | " | " | " | 95,7 |
| 144 | 2 | 2,8 | " | " | " | " | 95,5 |
| 144 | 2 | 1 | " | " | " | " | 91,3 |
| 65 | 1 | 2 | " | " | " | " | 95 |

Tableau II

| Solution de $Na^{131}I$ | | Solution d'acide gras dans l'acétone + NaI entraîneur | | | Chauffage | | Rendement de marquage |
|---|---|---|---|---|---|---|---|
| Volume (µl) | Activité totale (µCi) | Volume (ml) | Concentration en acide gras (mg/ml) | Rapport molaire acide gras/NaI | T(°C) | Durée (min.) | |
| 10 | 10 | 0,5 | 4 | $7,7.10^2$ | 99 | 5 | 95,4 |
| 20 | 20 | 0,5 | 4 | " | " | " | 94 |
| 25 | 25 | 0,5 | 4 | " | " | " | 92,3 |
| 30 | 30 | 0,5 | 4 | " | " | " | 90,9 |
| 40 | 40 | 0,5 | 4 | " | " | " | 86,9 |

**Revendications**

1. Procédé de préparation d'un acide gras marqué à l'iode radioactif, selon lequel on fait réagir un acide gras bromé ou iodé, de préférence en position oméga, avec de l'iodure radioactif pour échanger par de l'iode radioactif le brome ou l'iode dudit acide gras, caractérisé en ce que l'on utilise de l'iodure radioactif à l'état sec ou une solution aqueuse d'iodure radioactif et en ce que l'on réalise la réaction en présence d'un iodure entraîneur.

2. Procédé selon la revendication 1, caractérisé en ce que l'iodure radioactif à l'état sec ou la solution aqueuse d'iodure radioactif a un pH d'environ 7.

3. Procédé selon l'une quelconque des revendications 1 et 2, caractérisé en ce que l'iodure entraîneur est de l'iodure de sodium.

4. Procédé selon l'une quelconque des revendications 1 à 3, caractérisé en ce que le produit de départ est un acide gras iodé.

5. Procédé selon l'une quelconque des revendications 1 à 4, caractérisé en ce que l'on dissout l'acide gras bromé ou iodé dans une cétone miscible à l'eau contenant l'iodure entraîneur et en ce que l'on ajoute ladite solution d'acide gras à l'iodure radioactif à l'état sec ou à la solution aqueuse d'iodure radioactif.

6. Procédé selon l'une quelconque des revendications 1 à 5, caractérisé en ce que l'on utilise une solution aqueuse d'iodure radioactif ayant une activité volumique d'au moins 20 millicuries par millilitre.

7. Procédé selon l'une quelconque des revendications 1 à 5, caractérisé en ce que l'on utilise de l'iodure radioactif à l'état sec ayant une activité telle que le rapport de son activité en millicurie au poids en milligramme de l'acide gras bromé ou iodé soit au plus égal à 0,6.

8. Procédé selon l'une quelconque des revendications 1 à 7, caractérisé en ce que l'iode radioactif est l'iode 123.

9. Procédé selon l'une quelconque des revendications 1 à 8, caractérisé en ce qu'après la réaction d'échange, on évapore la cétone puis on ajoute une solution tampon injectable légèrement alcaline pour former une suspension colloïdale d'acide gras marqué et on dissout ladite suspension dans un liquide compatible avec le corps humain.

10. Procédé selon la revendication 9, caractérisé en ce que le liquide est de la sérum albumine humaine.

11. Procédé selon l'une quelconque des revendications 1 à 10, caractérisé en ce que l'acide gras de départ est un acide gras iodé répondant à la formule :

$$I(CH_2)_{n'-1}—Y—(CH_2)_{n-1}—COOH$$

dans laquelle Y représente les enchaînements $—CH_2—CH_2—$ ou $—CH{=}CH—$ Z ou E et n et n' sont des nombres entiers de 3 à 10, et en ce que l'on prépare ledit acide gras iodé par un procédé consistant à condenser un acide oméga bromé de formule :

$$Br—(CH_2)_{n-1}—COOH$$

et un alcool acétylénique de formule :

$$H—C{\equiv}C—(CH_2)_{n'}—CH_2OH,$$

dans l'ammoniac liquide en présence d'amidure de lithium pour obtenir l'acide hydroxy-oméga-alcynoïque de formule :

$$HO—(CH_2)_{n'-1}—C{\equiv}C—(CH_2)_{n-1}—COOH,$$

à soumettre ensuite à une réduction l'acide hydroxy-oméga-alcynoïque pour obtenir l'acide hydroxy-oméga-alcénoïque ou l'acide hydroxy-alcanoïque correspondant, à faire réagir l'acide hydroxy-oméga-alcanoïque ou l'acide hydroxy-oméga-alcénoïque ainsi obtenu avec du chlorure de paratoluène sulfonyle pour obtenir le dérivé tosylé d'acide gras correspondant, et à soumettre le dérivé tosylé ainsi obtenu à une réaction d'échange tosylate-iode pour obtenir le dérivé iodé correspondant.

12. Procédé selon la revendication 11, caractérisé en ce que l'on réalise la réduction partielle de l'acide hydroxy-oméga-alcynoïque en acide hydroxy-ω-alcénoïque Z par hydrogénation en présnece de quinoline et au moyen d'un catalyseur à 5 % de palladium sur du carbonate de calcium qui a été désactivé à 95 °C par une solution d'acétate de plomb.

13. Procédé selon la revendication 11, caractérisé en ce que l'on réalise la réduction partielle de l'acide hydroxy-oméga-alcynoïque en acide hydroxy- -alcénoïque E au moyen de sodium en solution dans l'ammoniac liquide, sous pression et à une température de 20 à 60 °C.

14. Procédé selon la revendication 11, caractérisé en ce que l'on réduit totalement l'acide hydroxy-oméga-alcynoïque pour obtenir l'acide hydroxy-oméga-alcanoïque correspondant par hydrogénation en présence d'un catalyseur au platine.

15. Procédé selon l'une quelconque des revendications 1 à 10, caractérisé en ce que le produit de départ est un acide gras iodé répondant à la formule :

$$I(CH_2)_{n'+1}—C \equiv C—(CH_2)_{n+1}—COOH$$

dans laquelle n et n' sont des nombres entiers de 3 à 10, et en ce que l'on prépare ledit acide gras iodé par un procédé consistant à condenser un acide gras bromé de formule :

$$Br—(CH_2)_{n+1}—COOH$$

et un alcool acétylénique de formule :

$$H—C \equiv C—(CH_2)_{n'}—CH_2OH$$

dans l'ammoniac liquide en présence d'amidure de lithium pour obtenir l'acide hydroxy-alcynoïque de formule :

$$HO—(CH_2)_{n'+1}—C \equiv C—(CH_2)_{n+1}—COOH$$

à faire réagir l'acide hydroxy-alcynoïque ainsi obtenu avec du chlorure de paratoluène sulfonyle pour obtenir le dérivé tosylé correspondant et à soumettre le dérivé tosylé ainsi obtenu à réaction d'échange tosylate-iode pour obtenir ledit acide gras iodé.

16. Procédé selon l'une quelconque des revendications 11 à 15, caractérisé en ce que l'on purifie l'acide gras iodé obtenu par chromatographie liquide à haute performance en utilisant un support à base de silice et un éluant constitué essentiellement par de l'heptane, de l'éther et de l'acide acétique.

**Claims**

1. A process for the production of a fatty acid labeled with radioactive iodine, in which a brominated or iodinated fatty acid (preferably at the omega position) is reacted with a radioactive iodide to exchange the bromine or iodine in said fatty acid with radioactive iodine, characterised in that the radioactive iodide is employed in the dry state or as an aqueous solution of radioactive iodide; and characterised in that the reaction is carried out in the presence of an entraining iodide.

2. A process according to Claim 1, characterised in that the radioactive iodide in the dry state, or the aqueous solution of radioactive iodide has a pH of about 7.

3. A process according to either of Claims 1 and 2, characterised in that the carrier iodide is sodium iodide.

4. A process according to any one of Claims 1 to 3, characterised in that the starting material is an iodinated fatty acid.

5. A process according to any one Claims 1 to 4, characterised in that the brominated or iodinated fatty acid is dissolved in a water-miscible ketone containing the carrier iodide, and in that said fatty acid solution is added to the radioactive iodide in the dry state or as an aqueous solution of radioactive iodide.

6. A process according to any one of Claims 1 to 5, characterised in that an aqueous radioactive iodide solution having an activity of at least 20 millicuries per millilitre, is employed.

7. A process according to any one of Claims 1 to 5, characterised in that the radioactive iodide is employed in the dry state having an activity such that the ratio of its activity in millicuries to the weight in milligrams of brominated or iodinated fatty acid is at least equal to 0.6.

8. A process according to any one of Claims 1 to 7, characterised in that the radioactive iodine is iodine − 123.

9. A process according to any one of Claims 1 to 8, characterised in that, after the exchange reaction, the ketone is evaporated and then a slightly alkaline buffer solution is added to form a colloïdal suspension of labelled fatty acid, and said suspension is dissolved in a liquid compatible with the human body.

10. A process according to Claim 9, characterised in that the liquid is human serum albumin.

11. A process according to any one of Claims 1 to 10, characterised in that the starting fatty acid is an iodinated fatty acid having the formula

$$I(CH_2)_{n'+1}—Y—(CH_2)_{n+1}—COOH$$

wherein Y represents a chain $—CH_2—CH_2—$ or $—CH = CH—$ (Z) or (E) and n and n' are integers from 3 to 10, and in that said iodinated fatty acid is prepared by a process comprising condensing an omega-bromo acid of the formula

$$Br—(CH_2)_{n+1}—COOH$$

and an acetylenic alcohol of the formula :

$$H—C≡C—(CH_2)_{n'}—CH_2OH,$$

in liquid ammonia in the presence of lithium amide, to obtain the hydroxy-omega-alkynoic acid of the formula :

$$HO—(CH_2)_{n'+1}—C≡C—(CH_2)_{n+1}—COOH,$$

and subsequently reducing the hydroxy-omega-alkynoic acid to obtain a corresponding hydroxy-omega-alkenoic acid or omega-hydroxy-alkanoic acid, and reacting the omega-hydroxy-alkanoic or omega-hydroxy-alkenoic acid thereby obtained with para-toluene suphonyl chloride to obtain the corresponding tosylated derivative of the fatty acid, and subjecting the tosylated derivative thereby obtained to a tosylate-iodine exchange reaction to provide the corresponding iodinated derivative.

12. A process according to Claim 11, characterised in that partial reduction of the omega-hydroxy alkynoic acid to omega-hydroxy alkenoic acid (Z) is achieved by hydrogenation in the presence of quinoline, and by means of a 5 % palladium catalyst on calcium carbonate, deactivated at 95 °C by a lead acetate solution.

13. A process according to Claim 11, characterised in that the partial reduction of the omega-hydroxy alkynoic acid to omega-hydroxy alkenoic acid (E) is obtained by means of sodium in liquid ammonia, under pressure at a temperature from 20 to 60 °C.

14. A process according to Claim 11, characterised in that the omega-hydroxy alkynoic acid is totally reduced to obtain the corresponding omega-hydroxy alkanoic acid by hydrogenation in the presence of a platinum catalyst.

15. A process according to any one of Claims 1 to 10, characterised in that the starting material is an iodinated fatty acid having the formula :

$$I(CH_2)_{n'+1}—C = C—(CH_2)_{n+1}—COOH$$

wherein n and n' are integers from 3 to 10, and in that said iodinated fatty acid is prepared by a process comprising condensing a brominated fatty acid of the formula :

$$Br—(CH_2)_{n+1}—COOH$$

and an acetylenic alcohol of the formula :

$$H—C≡C—(CH_2)_{n'}—CH_2OH$$

in liquid ammonia in the presence of lithium amide to obtain the hydroxy-alkynoic acid of the formula :

$$HO—(CH_2)_{n'+1}—C≡C—(CH_2)_{n+1}—COOH$$

reacting the hydroxy-alkynoic acid thereby obtained with paratoluene sulfonyl chloride to obtain the corresponding tosylated derivative, and subjecting the tosylated derivative thereby obtained to a tosylate-iodine exchange reaction to provide said iodinated fatty acid.

16. A process according to any one of Claims 11 to 15, characterised in that the iodinated fatty acid obtained is purified by high performance liquid chromatography, employing a substrate based on silica, and an eluant comprising heptane, ether, and acetic acid.

**Patentansprüche**

1. Verfahren zur Herstellung einer mit radioaktivem Jod markierten Fettsäure, bei dem man eine vorzugsweise in ω-Stellung bromierte oder jodierte Fettsäure mit radioaktivem Jodid reagieren läßt, um das Brom oder das Jod der Fettsäure durch das radioaktive Jod auszutauschen, dadurch gekennzeichnet, daß man radioaktives Jodid im trockenen Zustand oder eine wässrige Lösung von radioaktivem Jodid verwendet und daß man die Reaktion in Gegenwart eines Jodid-Trägers durchführt.

2. Verfahren nach Anspruch 1, dadurch gekennzeichnet, daß das radioaktive Jodid im trockenen Zustand oder die wässrige Lösung von radioaktivem Jodid einen pH-Wert von ungefähr 7 aufweist.

3. Verfahren nach Anspruch 1 oder 2, dadurch gekennzeichnet, daß der Jodid-Träger Natrium-Jodid ist.

4. Verfahren nach einem der Ansprüche 1 bis 3, dadurch gekennzeichnet, daß das Ausgangsprodukt eine jodierte Fettsäure ist.

5. Verfahren nach einem der Ansprüche 1 bis 4, dadurch gekennzeichnet, daß man die bromierte oder jodierte Fettsäure in einem Keton auflöst, welches mit dem den Jodid-Träger enthaltenden Wasser

vermischbar ist, und daß man diese Fettsäurelösung dem radioaktiven Jodid im trockenen Zustand oder der wässrigen Lösung von radioaktivem Jodid hinzufügt.

6. Verfahren nach einem der Ansprüche 1 bis 5, dadurch gekennzeichnet, daß man eine wässrige Lösung von radioaktivem Jodid verwendet, die eine Volumenaktivität von wenigstens 20 Millicurie pro Milliliter aufweist.

7. Verfahren nach einem der Ansprüche 1 bis 5, dadurch gekennzeichnet, daß man radioaktives Jodid im trockenen Zustand verwendet, welches eine solche Aktivität aufweist, daß das Verhältnis seiner Aktivität in Millicurie zum Gewicht in Milligramm der bromierten oder jodierten Fettsäure höchstens gleich 0,6 ist.

8. Verfahren nach einem der Ansprüche 1 bis 7, dadurch gekennzeichnet, daß das radioaktive Jod das Jod 123 ist.

9. Verfahren nach einem der Ansprüche 1 bis 8, dadurch gekennzeichnet, daß man nach der Austauschreaktion das Keton verdampft und dann eine leicht alkalische, injizierbare Pufferlösung hinzugibt, um eine kolloidale Suspension von markierter Fettsäure zu bilden, und daß man die Suspension in einer mit dem menschlichen Körper verträglichen Flüssigkeit auflöst.

10. Verfahren nach Anspruch 9, dadurch gekennzeichnet, daß die Flüssigkeit menschliches Albumin-Serum ist.

11. Verfahren nach einem der Ansprüche 1 bis 10, dadurch gekennzeichnet, daß die Ausgangsfettsäure eine jodierte Fettsäure nach der Formel ist :

$$I(CH_2)_{n'+1}—Y—(CH_2)_{n+1}—COOH$$

wobei in der Formel Y die Kettenbildung $—CH_2—CH_2—$ oder $—CH = CH—$ Z oder E darstellt und n und n' ganze Zahlen von 3 bis 10 sind, und daß man die genannte jodierte Fettsäure nach einem Verfahren herstellt, welches darin besteht, daß man eine in der Omega-Stellung bromierte Säure der Formel :

$$Br—(CH_2)_{n+1}—COOH$$

und einen acetylenischen Alkohol der Formel

$$H—C \equiv C—(CH_2)_{n'}—CH_2OH$$

in flüssigem Ammoniak in Gegenwart von Lithiumamid kondensiert, um eine Hydroxy-Omega-Alkinsäure der Formel

$$HO—(CH_2)_{n'+1}—C \equiv C(CH_2)_{n+1}—COOH$$

zu erhalten, daß man daraufhin die Hydroxy-Omega-Alkinsäure reduziert, um die entsprechende Hydroxy-Omega-Alkensäure oder Hydroxy-Alkansäure zu erhalten, daß man die derart erhaltene Hydroxy-Omega-Alkansäure oder die Hydroxy-Omega-Alkensäure mit p-Toluolsulfonylchlorid reagieren läßt, um die entsprechende Tosyl-Fettsäure zu erhalten, und daß man die derart erhaltene Tosyl-Verbindung einer Tosylat-Jod-Austauschreaktion aussetzt, um die entsprechende jodierte Verbindung zu erhalten.

12. Verfahren nach Anspruch 11, dadurch gekennzeichnet, daß man die teilweise Reduzierung der Hydroxy-Omega-Alkinsäure zu Hydroxy-Omega-Alkensäure Z durch Hydrierung in Gegenwart von Chinolin und wenigstens einem Katalysator von 5 % Palladium auf Kalziumkarbonat durchführt, welcher bei 95 °C durch eine Bleiacetatlösung desaktiviert worden ist.

13. Verfahren nach Anspruch 11, dadurch gekennzeichnet, daß man die teilweise Reduzierung der Hydroxy-Omega-Alkinsäure zu Hydroxy-Omega-Alkensäure E mittels in flüssigem Ammoniak gelöstem Natrium unter Druck und bei einer Temperatur von 20 bis 60 °C durchführt.

14. Verfahren nach Anspruch 11, dadurch gekennzeichnet, daß man die Hydroxy-Omega-Alkinsäure durch Hydrierung in Gegenwart eines Platin-Katalysators vollständing reduziert, um die entsprechende Hydroxy-Omega-Alkansäure zu erhalten.

15. Verfahren nach einem der Ansprüche 1 bis 10, dadurch gekennzeichnet, daß das Ausgangsprodukt eine jodierte Fettsäure ist, die die Formel erfüllt :

$$I(CH_2)_{n'+1}—C \equiv C—(CH_2)_{n+1}—COOH$$

in der n und n' ganze Zahlen von 3 bis 10 sind, und daß man die jodierte Fettsäure nach einem Verfahren herstellt, welches darin besteht, eine bromierte Fettsäure der Formel

$$Br—(CH_2)_{n+1}—COOH$$

und einen acetylenischen Alkohol der Formel

$$H—C \equiv C—(CH_2)_{n'}—CH_2OH$$

in flüssigem Ammoniak in Gegenwart von Lithiumamid zu kondensieren, um eine Hydroxy-Alkinsäure der Formel

$$HO-(CH_2)_{n'+1}-C\equiv C-(CH_2)_{n+1}-COOH$$

zu erhalten, daß man die derart erhaltene Hydroxy-Alkinsäure mit p-Toluolsulfonylchlorid reagieren läßt, um die entsprechende Tosyl-Verbindung zu erhalten, und daß man die derart erhaltene Tosyl-Verbindung einer Tosylat-Jod-Austauschreaktion aussetzt, um die jodierte Fettsäure zu erhalten.

16. Verfahren nach einem der Ansprüche 11 bis 15, dadurch gekennzeichnet, daß man die erhaltene, jodierte Fettsäure durch Hochleistungs-Flüssigchromatographie reinigt, indem man einen Träger auf Siliciumoxydbasis und ein Eluierungsmittel verwendet, welches im wesentlichen aus Heptan, Äther und Essigsäure besteht.